# EUROPEAN PATENT APPLICATION

(11) **EP 4 786 471 A1**
(43) Date of publication of application: **05.08.2026**
(21) Application number: 24870932.1
(22) Date of filing: 27.09.2024
(51) Int. Cl.: C07D 491/22, A61K 31/4745, A61P 35/00, A61K 47/68

(54) **HALOGEN-SUBSTITUTED EXATECAN AMIDE DERIVATIVE, PREPARATION METHOD THEREFOR, AND USE THEREOF**

(30) Priority: 28.09.2023 CN 202311289220
(71) Applicant: Hangzhou Adcoris Biopharma Co., Ltd, Hangzhou, Zhejiang 310052 (CN)
(72) Inventor: MIAO, Zhenwei, Hangzhou, Zhejiang 310052 (CN); HUANG, Yunsheng, Hangzhou, Zhejiang 310052 (CN)
(74) Representative: Elzaburu S.L.P.
(86) International application number: PCT/CN2024/121636
(87) International publication number: WO 2025/067394

(57) **Abstract**

The present invention relates to the field of biological medicines, and provides a halogen-substituted exatecan amide derivative, a preparation method therefor, and a use thereof; the halogen-substituted exatecan amide derivative or a pharmaceutically acceptable salt, stereoisomer, or prodrug thereof has good cancer cell inhibitory activity and activity across the cell membrane, and can be applied to the treatment of cancer as a single drug, a combined drug, or a toxin component of an ADC.

## Description

The present application claims priority from the Chinese patent application No. 2023112892206 filed on September 28, 2023, and the present application incorporates by reference the entire content of the above-mentioned Chinese patent application.

### TECHNICAL FIELD

The present disclosure relates to the field of biomedicine, and specifically relates to halogen-substituted exatecan amide derivatives, preparation method, and use thereof.

### BACKGROUND

Exatecan is an derivative of the natural product camptothecin, and has inhibitory activity against topoisomerase Top1, particularly exhibiting strong inhibitory activity against the complex formed by Top1-DNA. Camptothecin shows significant efficacy against gastric cancer, esophageal cancer, lung cancer, bladder cancer, etc., and is a broad-spectrum antitumor agent. Among them, Irinotecan and Topotecan have been approved for the treatment of various cancers in many countries.

The camptothecin derivative Belotecan has been approved in South Korea for the treatment of SCLC and ovarian cancer. The main drawbacks of camptothecin antitumor drugs are their toxicity, poor solubility, and the development of drug resistance in tumor cells.

### SUMMARY

In order to solve the above-mentioned problems, the present disclosure provides a halogen-substituted exatecan amide derivative, and a preparation method and use thereof. The derivative has good cancer cell inhibitory activity and activity across the cell membrane, and can be applied to the treatment of cancer as a single drug, a combined drug, or a toxin component of an ADC.

One aspect of the present disclosure provides a compound of formula (I), or a pharmaceutically acceptable salt, a stereoisomer, or a prodrug thereof: 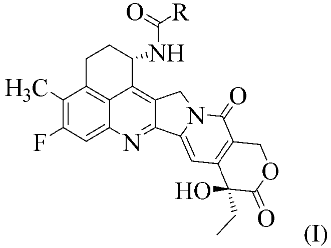
in the Formula, R is selected from any one of C₁~C₆ alkyl, halogen-substituted C₁~C₆ alkyl, 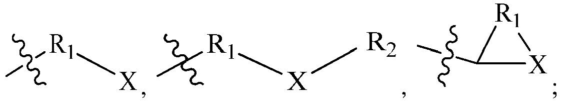
wherein, R₁ is selected from C₁~C₆ alkylene, the C₁~C₆ alkylene is optionally substituted with one or more substituents selected from halogen, C₁~C₆ alkyl or halogen-substituted C₁~C₆ alkyl;
R₂ is selected from any one of halogen, C₁~C₆ alkyl or halogen-substituted C₁~C₆ alkyl; X is selected from any one of amino, imino.

In some embodiments of the present disclosure, R₁ is selected from methylene, halogen-substituted methylene or trifluoromethyl-substituted methylene.

In some embodiments of the present disclosure, R₁ is selected from -CHF-, -CHF₂-, -CH₂- or - CH(CF₃)-.

In some embodiments of the present disclosure, R₂ is selected from any one of halogen, methyl, methylene, ethyl, propyl, isopropyl, butyl, isobutyl, pentyl, isopentyl, hexyl, heptyl; preferably, any one of methylene, methyl, ethyl, isopropyl.

The halogen is selected from any one of -F, -Cl, -Br, or -I, preferably -F.

In some embodiments of the present disclosure, X is selected from -NH- or -NH₂.

In some embodiments of the present disclosure, 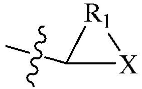 is selected from 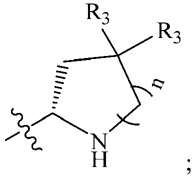
wherein, R₁ is selected from halogen; preferably F;
n is selected from 1, 2, 3 or 4, preferably, n is 1.

In some embodiments of the present disclosure, R is fluoro-substituted. It exhibits superior tumor cell inhibitory activity and cell membrane permeability.

In some embodiments of the present disclosure, R is selected from

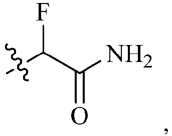

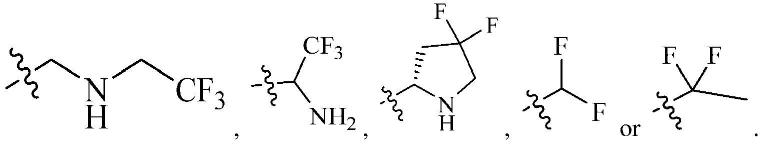

In some embodiments of the present disclosure, the above-mentioned compound, or pharmaceutically acceptable salt, stereoisomer, or prodrug thereof, wherein, the compound of formula (I) is any one of the following compounds:

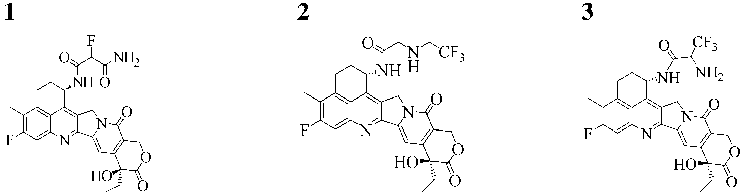

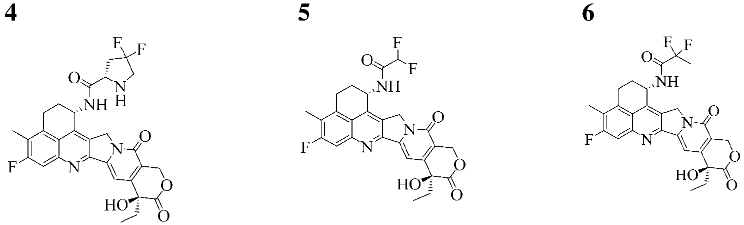

As another aspect of the present disclosure, it provides a method for preparing the above-mentioned compound, comprising: reacting halogen-substituted carboxylic acid, carboxylic anhydride, acyl chloride with exatecan to prepare the above-mentioned compound; preferably, the above-mentioned halogen-substituted halogen-substituted carboxylic acid, carboxylic anhydride, or acyl chloride is selected from any one of 2-fluoromalonic acid, 2-amino-3,3,3-trifluoropropanoic acid, difluoroacetic anhydride, and 2,2-difluoropropanoic acid.

In some embodiments of the present disclosure, wherein, the halogen-substituted carboxylic acid, carboxylic anhydride, or acyl chloride further comprises amino-protected halogen-substituted carboxylic acid, carboxylic anhydride, or acyl chloride; exatecan further comprises the corresponding salts of exatecan; and the reaction is carried out directly or in the presence of a condensing agent.

In some embodiments of the present disclosure, the amino protecting group in the amino protection method is selected from any one of benzyloxycarbonyl (Cbz-), tert-butoxycarbonyl (Boc-); the amino-protected halogen-substituted carboxylic acid, carboxylic anhydride, acyl chloride is selected from any one of N-Cbz-N-trifluoroethylglycine ( 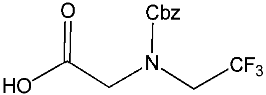 ), N-Boc-4,4-difluoroproline ( 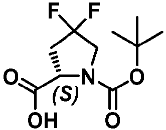 ); the corresponding salt of exatecan is selected from exatecan mesylate; and the condensing agent is any one of N,N-diisopropylcarbodiimide (DIC), 1-ethyl-(3-dimethylaminopropyl)carbodiimide (EDC).

As a further aspect of the present disclosure, it provides an antibody-drug conjugate, comprising a small molecule drug, a linker, and an antibody, wherein, the above-mentioned small molecule drug comprises the above-mentioned compound, or pharmaceutically acceptable salt, stereoisomer, or prodrug thereof.

As a further aspect of the present disclosure, it provides a pharmaceutical composition, comprising the above-mentioned compound, or pharmaceutically acceptable salt, stereoisomer, or prodrug thereof.

As a further aspect of the present disclosure, it provides a use of the above-mentioned compound, or pharmaceutically acceptable salt, stereoisomer, or prodrug thereof, the compound afforded by the above-mentioned preparation method, the above-mentioned antibody-drug conjugate, or the above-mentioned pharmaceutical composition, in the manufacture of a medicament for treating a cancer.

As a further aspect of the present disclosure, it provides a method for treating a cancer, comprising the step of administering to a patient in need thereof the above-mentioned compound, or pharmaceutically acceptable salt, stereoisomer, or prodrug thereof, the compound afforded by the above-mentioned preparation method, the above-mentioned antibody-drug conjugate, or the above-mentioned pharmaceutical composition.

Further, comprising the step of administering to a patient in need thereof a therapeutically effective amount of the above-mentioned compound.

In some embodiments of the present disclosure, the cancer comprises any one of gastric cancer, esophageal cancer, breast cancer, breast adenocarcinoma, lung cancer.

In some embodiments of the present disclosure, the cancer cells are selected from any one of OE33 cells (human esophageal cancer cells), MDA-MB-231 (human breast cancer cells), NCI-N87 (human gastric cancer cells), NCI-H1975 (human lung cancer cells), and TE12 (human esophageal cancer cells).

### DETAILED DESCRIPTION OF THE EMBODIMENTS

### I. Definition

In the present disclosure, unless otherwise specified, scientific and technical terms used herein have the meanings commonly understood by those skilled in the art. Also, the relevant terms and laboratory procedures used herein are terms and conventional procedures widely used in the corresponding fields. Meanwhile, for a better understanding of the present disclosure, definitions and explanations of related terms are provided below.

As used herein and unless otherwise specified, the terms "comprising", "including", "having", "containing", including grammatical equivalents thereof, should generally be understood to be openended and non-limiting, for example, not excluding other unrecited elements or steps.

The compound of the present disclosure can be asymmetric, for example, having one or more stereoisomers. Unless otherwise specified, all stereoisomers are included, for example, enantiomers and diastereomers. The stereoisomers include geometric isomers (such as cis and trans structures) and optical isomers (such as enantiomers), therapeutic substances consisting of monomers, racemates, racemic mixtures and pharmaceutically acceptable salts thereof. The compound of the present disclosure containing asymmetric carbon atoms can be isolated in optically active pure form or racemic form. Optically active pure forms can be resolved from racemic mixtures or synthesized by using chiral starting materials or chiral reagents. Racemates, diastereomers, enantiomers are all included within the scope of the present disclosure.

The compound of the present disclosure also includes tautomeric forms. Tautomeric forms arise from the exchange of one single bond with an adjacent double bond and together with the transfer of one proton.

As used herein, "pharmaceutically acceptable salt" refers to the salt of the corresponding amine compound with an inorganic or organic acid, or the salt of the corresponding carboxylic acid compound with an alkali metal or alkaline earth metal, or with an organic amine. Wherein, inorganic acids include but are not limited to hydrochloric acid, hydrobromic acid, hydroiodic acid, sulfuric acid, phosphoric acid, etc; organic acids include but are not limited to acetic acid, propionic acid, butyric acid, benzoic acid, methanesulfonic acid, toluenesulfonic acid, p-toluenesulfonic acid, oxalic acid, succinic acid, lactic acid, citric acid, succinic acid, gluconic acid, maleic acid, fumaric acid, tartaric acid, etc; alkali metal or alkaline earth metal salts include but are not limited to sodium, potassium, calcium, magnesium salts, etc; organic amine salts include, but are not limited to, salts composed of ammonia, methylamine, ethylamine, propylamine, isopropylamine, dimethylamine, diethylamine, trimethylamine, triethylamine, tert-butylamine, ethylenediamine, ethanolamine, diethanolamine, triethanolamine, morpholine, piperidine, piperazine, amino acids, etc.

As used herein, "prodrug" refers to the form in which a compound is metabolized or simply chemically altered in the body of a patient after the compound has been introduced into the human body in a suitable mode of administration to the compound contained in general formula 1 of the present invention, as well as the corresponding salt. Prodrugs of the compounds include, but are not limited to, various carboxylate esters, carbonate esters, phosphate esters, sulfate esters, sulfonate esters, amino acid esters, gluconate esters, as well as various amides, acetals, hemiacetals, carbamide esters, etc.

The numerical range as used herein refers to each integer within the given range. For example,"C₁-C₆" refers to the group that can have 1 carbon atom, 2 carbon atoms, 3 carbon atoms, 4 carbon atoms, 5 carbon atoms or 6 carbon atoms; "C₃-C₆" refers to the group that can have 3 carbon atoms, 4 carbon atoms, 5 carbon atoms or 6 carbon atoms.

When any variable (such as Rn) appears more than once in the composition or structure of a compound, the definition thereof is independent in each case. Therefore, for example, if a group is substituted with 1-5 R's, then the group can optionally be substituted with up to 5 R's, and each R has independent options in every case. In addition, combinations of substituents and/or variants thereof are only allowed if such combinations result in the formation of stable compounds.

The term "alkyl" refers to a saturated aliphatic hydrocarbon group, which is an alkyl containing 1 to 6 carbon atoms, preferably an alkyl containing 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl. More preferably, is a lower alkyl containing 1 to 6 carbon atoms, non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. The alkyl can be substituted or unsubstituted, and when substituted, substituents can be substituted at any available point of attachment, the substituents preferably being one or more of the following groups, which are independently selected from alkyl, halogen, with methyl, ethyl, isopropyl, tert-butyl, halogenated alkyl being preferred for the present disclosure.

The hydrogen atoms of the present disclosure can be substituted by the isotope deuterium thereof.

The term "substituted" refers to one or more hydrogen atoms in a group, preferably up to 5, and more preferably 1-3 hydrogen atoms, being independently substituted with the corresponding number of substituents. It goes without saying that the substituents are only in their possible chemical positions, and those skilled in the art can determine (through experiment or theory) which substitutions are possible or impossible without undue effort. For example, combinations such as an amino or hydroxy having a free hydrogen with a carbon atom having an unsaturated (e.g., olefinic) bond may be unstable. " 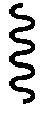 " refers to the chemical bond junction.

### Medicament or pharmaceutical composition

The drugs or drug compositions of the present disclosure can be administered orally, topically, parenterally, or mucosally (e.g., sublingually, by inhalation, or rectally) in dose unit formulations comprising conventional non-toxic pharmaceutically acceptable carriers. An oral route is generally preferred. The active agents can be administered orally in the form of capsules, tablets, etc. (see Remington: The Science and Practice of Pharmacy, 20th Edition).

For oral administration in the form of tablets or capsules, the active pharmaceutical ingredients can be combined with non-toxic, pharmaceutically acceptable excipients such as binders (for example, pregelatinized corn starch, polyvinyl pyrrolidone, or hydroxypropyl methylcellulose); fillers (for example, lactose, sucrose, glucose, mannitol, sorbitol, and other reducing and non-reducing sugars, microcrystalline cellulose, calcium sulfate, or calcium hydrogen phosphate); lubricants (for example, magnesium stearate, talc, or silica, stearic acid, sodium stearyl fumarate, glyceryl behenate, calcium stearate, etc.); disintegrants (for example, potato starch or sodium starch glycolate); or wetting agents (for example, sodium lauryl sulfate), colorants and flavorings, gelatin, sweeteners, natural and synthetic gums (such as acacia gum, tragacanth gum, or alginates), buffering salts, carboxylmethyl cellulose, polyethylene glycol, waxes, etc. For oral administration in liquid form, the pharmaceutical ingredients can be combined with non-toxic, pharmaceutically acceptable inert carriers (for example, ethanol, glycerin, water), anti-sedimentation agents (for example, sorbitol syrup, cellulose derivatives, or hydrogenated edible fats), emulsifiers (for example, lecithin or acacia gum), non-aqueous carriers (for example, almond oil, oil esters, ethanol, or fractionated vegetable oils), preservatives (for example, methyl p-hydroxybenzoate or propyl p-hydroxybenzoate or sorbic acid), etc. Stabilizers such as antioxidants (BHA, BHT, propyl gallate, sodium ascorbate, citric acid) can also be added to stabilize the dosage form.

Tablets containing the active compound can be coated using methods well-known in the field. The compositions of the present disclosure, which include the compound represented by formula I as the active compound, can also be introduced into small beads, microspheres, or microcapsules, for example, constructed with polyglycolic acid/lactic acid (PGLA). Liquid formulations for oral administration can take the forms of e.g. solution, syrup, emulsion, or suspension, or they can be presented as dry products that are reconstituted with water or other suitable excipients before use. Formulations for oral administration can be suitably formulated to provide controlled or delayed release of the active compound.

The term "treatment" includes inhibiting, alleviating, preventing, or eliminating one or more symptoms or side effects associated with the disease, condition, or disorder being treated.

The term "inhibition" is used in relation to controls. Those skilled in the art will readily determine the appropriate control for each experiment. For example, a reduced response in subjects or cells treated with the compound is compared to the response in subjects or cells not treated with the compound.

The term "pharmaceutical composition" refers to a composition that includes the compound or pharmaceutically acceptable salt thereof of the present disclosure, as well as at least one pharmaceutically acceptable component selected from the following, depending on the administration route and the nature of dosage form, including but not limited to: carriers, diluents, adjuvants, excipients, preservatives, fillers, disintegrants, wetting agents, emulsifiers, suspension agents, sweeteners, flavoring agents, fragrances, antimicrobials, antifungals, lubricants, dispersing agents, temperature-sensitive materials, temperature regulators, adhesives, stabilizers, suspending agents, etc.

The term "effective amount" or "therapeutically effective amount" refers to a sufficient amount of a drug or medication that is non-toxic but can achieve the desired effect. In embodiments of the present disclosure, when treating a patient in accordance with the present disclosure, the amount of drugs administered depends on many factors, such as the specific dosing regimen, the type of disease or condition and the severity thereof, the uniqueness (for example, body weight) of the treated person or host to be treated, but the amount of drug administered depends on specific surrounding circumstances, including, for example, the specific drug that has been employed, the route of administration, the treated condition, and the treated person or host to be treated, the administered dose may be routinely determined by methods known in the art. Generally, regarding the dosage used for adult therapy, the administered dose is typically within the range of 0.02-5000mg/day, for example, the range of about 1-1500mg/day. The required dose can be conveniently administered as a single dose, or as doses given simultaneously (or in a short period of time) or in divided doses at appropriate intervals, such as two, three, four doses per day or more. It can be understood by those skilled in the art that, although the above dose ranges are provided, the specific effective amount can be appropriately adjusted based on the patient's condition and in conjunction with a physician's diagnosis.

The term "antibody-drug conjugate (ADC)" refers to a biologically active small molecule drug linked to a monoclonal antibody via a chemical linker. The monoclonal antibody serves as a carrier to target and deliver the small molecule drug to specific target cells.

As used herein, the terms "reduce", "inhibit", "alleviate", or "decrease" are used in relation to controls. Those skilled in the art will easily determine the appropriate control for each experiment. For example, a reduced response in subjects or cells treated with the compound is compared to the response in subjects or cells not treated with the compound.

Unless otherwise specified, the raw materials and equipment used in the specific embodiments of the present disclosure are known products that can be afforded by purchasing commercial products.

### Abbreviation

DIPEA: diisopropylethylamine
DMF: N,N-dimethylformamide
DIC: N,N-diisopropylcarbodiimide
DCM: dichloromethane
HATU: 2-(7-azabenzotriazol)-N,N,N',N'-tetramethyluronium hexafluorophosphate
N-Cbz-: N-benzyloxycarbonyl
TFA: trifluoroacetic acid
EDCI: 1-ethyl-(3-dimethylaminopropyl)carbodiimide hydrochloride
EDC: 1-ethyl-(3-dimethylaminopropyl)carbodiimide
NHS: N-hydroxysuccinimide
TEA: triethylamine

### II. Examples

### Example 1: N-(2'-fluoro-3'-amino-3'-oxo-propanoyl)exatecan (1)

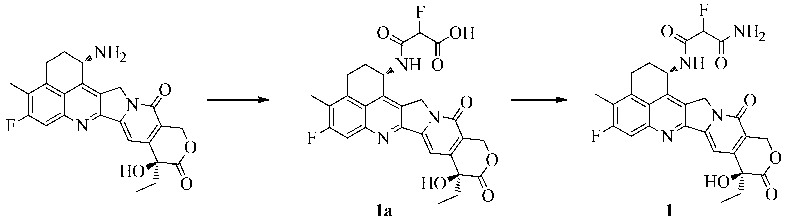

In a 50 mL single-necked flask, exatecan mesylate (265.8 mg, 0.5 mmol) and DIPEA (125.3 mg, 1.0 mmol) were dissolved in DMF (5 mL). 2-Fluoromalonic acid (183.1 mg, 1.5 mmol) and DIC (63.1 mg, 0.5 mmol) were dissolved in DCM (2.5 mL) and added to the single-necked flask. After being stirred to react at room temperature for 3 h, the mixture was purified by reversed-phase column chromatography to afford the yellow solid product **1a**, 2-fluoromonomalonyl exatecan (155 mg, yield 57%); LCMS: (M+1)⁺ 540.22 (calculated value: 539.49).

In a 10 mL single-necked flask, the above-mentioned intermediate **1a** (50 mg, 92.68 µmol) was dissolved in DMF (2 mL) and stirred until dissolved. HATU (70.5 mg, 185.5 µmol), DIPEA (48 mg, 371 µmol) and NH₄Cl (14.9 mg, 278 µmol) were added sequentially. The mixture was stirred to react at room temperature for 1 h, then purified by reversed-phase column chromatography to afford the yellow solid product **1**, N-(2'-fluoro-3'-amino-3'-oxo-propanoyl)exatecan (30.2 mg, yield 60.5%); ¹H NMR (500 MHz, DMSO-*d*₆) δ 9.05 (d, *J*=8.5 Hz, 1H), 7.81 (dt, *J*=11.2, 5.5 Hz, 2H), 7.69 (d, *J*=4.1 Hz, 1H), 7.32 (d, *J*=1.5 Hz, 1H), 6.52 (s, 1H), 5.60 (dt, *J*=8.3, 4.2 Hz, 1H), 5.43 (s, 2H), 5.38-5.24 (m, 2H), 5.21-5.05 (m, 2H), 3.18 (ddd, *J*=18.0, 11.1, 5.4 Hz, 2H), 2.47-2.35 (s, 3H), 2.30-2.19 (m, 1H), 2.18-2.07 (m, 1H), 1.95-1.80 (m, 2H), 0.87 (t, *J*=7.3 Hz, 3H); LCMS: (M+1)⁺ 539.28 (calculated value: 538.51).

### Example 2: N-(N-(2'-trifluoroethyl)aminoacetyl)exatecan (2)

Under ice-bath cooling, benzyl chloroformate (8.84 mL, 62.74 mmol), trifluoroethylamine (7.5 g, 57.03 mmol), Na₂CO₃ (9.07 g, 85.55 mmol) and dioxane:H₂O (1:1, 150 mL) were added to a reaction flask. The reaction mixture was stirred at room temperature overnight. The solvent was removed under reduced pressure, and the residue was extracted with methyl tert-butyl ether. After concentration, crude product **2a**, N-Cbz-trifluoroethylamine (9 g, crude yield 68%) was afforded, which was used directly in the next step without purification.

Under ice-bath cooling, N-Cbz-trifluoroethylamine **2a** (3.5 g, 14.94 mmol), DMF (30 mL), NaH (60% dispersion in oil, 597.74 mg, 14.94 mmol) and tert-butyl 2-bromoacetate (2.20 mL, 14.94 mmol) were added to a reaction flask. The mixture was stirred to react at room temperature for 3 hours. Aqueous ammonium chloride solution was added, and the mixture was extracted with methyl tert-butyl ether, purified by silica gel column chromatography to afford **2b**, tert-butyl N-Cbz-N-trifluoroethylglycinate (3.6 g, yield 69 %); ¹H NMR (600 MHz, CDCl₃) δ 7.35 (dq, *J*=18.0, 7.2, 6.8 Hz, 5H), 5.18 (d, *J*=10.1 Hz, 2H), 4.07-3.89 (m, 4H), 1.42 (d, *J*=44.5 Hz, 9H).

Under ice-bath cooling, **2b** (3.6 g, 10.33 mmol), DCM (30 mL) and TFA (6 mL) were added to a reaction flask. The mixture was stirred at room temperature overnight. The solvent was removed under reduced pressure, and the residue was extracted with methyl tert-butyl ether. The solvent was removed to afford the colorless oil **2c**, N-Cbz-N-trifluoroethylglycine (3.4 g, yield 81 %); ¹H NMR (600 MHz, DMSO-*d*₆) δ 7.47-7.20 (m, 2H), 5.13 (d, *J*=17.7 Hz, 1H), 4.17 (dq, *J*=18.7, 9.2 Hz, 1H), 4.03 (d, *J*=33.5 Hz, 1H).

To a reaction flask, **2c** (267.52 mg, 915.43 µmol), NHS (105.72 mg, 910.62 µmol), EDCI (176.10 mg, 918.60 µmol) and DCM (10 mL) were added. The mixture was stirred at room temperature for 1 hour. A solution of exatecan mesylate (0.25 g, 574.12 µmol) in DCM (5 mL) was added to the reaction mixture. The mixture was stirred at room temperature for 2 hours, then poured into water and extracted with DCM. The solvent was removed under reduced pressure, and purified by silica gel column chromatography to afford the white solid product **2d**, N-trifluoromethyl-N-Cbz-glycyl exatecan (300 mg, yield 73.5 %); ¹H NMR (600 MHz, CDCl₃) δ 7.62 (d, *J*=10.4 Hz, 1H), 7.53 (s, 1H), 7.18 (d, *J*=75.9 Hz, 6H), 6.73 (s, 1H), 5.65 (d, *J*=16.2 Hz, 1H), 5.56 (s, 1H), 5.16 (dt, *J*=19.9, 14.0 Hz, 4H), 5.08-4.99 (m, 1H), 4.33-4.13 (m, 2H), 4.05 (d, *J*=16.4 Hz, 2H), 3.96 (s, 1H), 3.04 (d, *J*=61.6 Hz, 2H), 2.40 (s, 3H), 2.26 (d, *J*=27.8 Hz, 2H), 1.96-1.84 (m, 2H), 1.05 (t, *J*=7.3 Hz, 3H). LCMS: m/z [M+H]⁺ 709.10 (calculated value 708.22).

To a reaction flask, **2d** (0.25 g, 352.28 µmol), THF (5 mL) and Pd-C (5%, 37.49 mg) were added. The mixture was reacted under a hydrogen balloon atmosphere and stirred overnight. The mixture was filtered, and the filtrate was concentrated to afford the solid product **2**, N-trifluoromethylglycyl exatecan (156 mg, yield 72 %); ¹H NMR (600 MHz, DMSO-*d*₆) δ 8.42 (d, *J*=8.8 Hz, 1H), 7.70 (d, *J*=10.8 Hz, 1H), 7.26 (s, 1H), 6.53 (s, 1H), 5.56 (dt, *J*=9.5, 5.0 Hz, 1H), 5.40 (s, 2H), 5.19-5.02 (m, 2H), 3.46-3.23 (m, 8H), 3.13 (dq, J=11.7, 5.7 Hz, 2H), 2.33 (s, 3H), 2.17 (tq, J=13.7, 7.7, 6.2 Hz, 2H), 1.85 (m, J=21.5, 7.3 Hz, 2H), 0.86 (t, J=7.3 Hz, 3H); ¹³C NMR (151 MHz, DMSO-*d*₆) δ 172.90, 171.24, 162.85, 161.20, 157.11, 152.75, 150.32, 148.34, 148.24, 145.61, 140.43, 136.61, 136.57, 127.57, 126.10, 125.72, 124.23, 124.10, 121.78, 119.60, 110.37, 110.22, 97.10, 72.79, 65.73, 51.97, 50.02, 49.96, 49.81, 49.61, 44.47, 40.39, 40.25, 40.11, 39.97, 39.83, 39.69, 39.56, 30.85, 28.03, 23.41, 11.36, 11.33, 8.19; LCMS: m/z [M+H]⁺ 575.26 (calculated value 574.18).

### Example 3: 2'-amino-3',3',3'-trifluoropropanoyl exatecan (3)

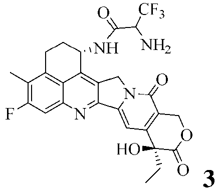

Under ice-bath cooling, 2-amino-3,3,3-trifluoropropanoic acid hydrochloride (1.08 g, 6.02 mmol), EDC hydrochloride (1.15 g, 6.02 mmol) and DMF (5 mL) were added to a reaction flask. The reaction mixture was stirred at 0 °C for 30 minutes, followed by stirring at room temperature for 1 hour. A solution of exatecan mesylate (2.0 g, 3.76 mmol) in DMF (80 mL) and triethylamine (609.17 mg, 6.02 mmol) were added to the reaction mixture. The reaction mixture was stirred at room temperature overnight. Water was added, and the mixture was extracted with DCM, dried, and purified by silica gel column chromatography to afford compound **3** as a brown solid, 2'-amino-3',3',3'-trifluoropropanoyl exatecan (1.2 g, yield 44.92%, HPLC 96%); ¹H NMR (600 MHz, DMSO-*d*₆) δ 9.41 (t, *J*=9.2 Hz, 1H), 7.86-7.76 (m, 1H), 7.30 (s, 1H), 6.53 (s, 1H), 5.61 (dt, *J*=8.3, 4.2 Hz, 1H), 5.47-5.28 (m, 4H), 4.93 (d, *J*=18.8 Hz, 1H), 4.58 (dd, *J*=15.0, 7.4 Hz, 1H), 3.32-3.03 (m, 3H), 2.39 (s, 3H), 2.35-2.25 (m, 1H), 2.21-2.12 (m, 1H), 1.86 (dh, *J*=20.8, 6.9 Hz, 2H), 0.87 (dt, *J*=13.8, 7.3 Hz, 3H); ¹³C NMR (151 MHz, DMSO-*d*₆) δ 172.84, 163.02, 162.64, 161.37, 157.15, 152.86, 150.44, 148.54, 148.44, 145.56, 138.57, 136.44, 126.55, 124.62, 124.49, 121.68, 121.50, 119.81, 110.61, 110.45, 97.33, 72.80, 65.71, 54.62, 49.49, 45.24, 44.30, 40.39, 40.25, 40.11, 39.97, 39.83, 39.69, 39.55, 30.81, 27.24, 27.06, 22.61, 11.43, 11.40, 8.23, 8.13; LCMS: m/z [M+H]⁺ 561.45 (calculated value 560.17).

### Example 4: 4',4'-difluoroprolyl exatecan (4)

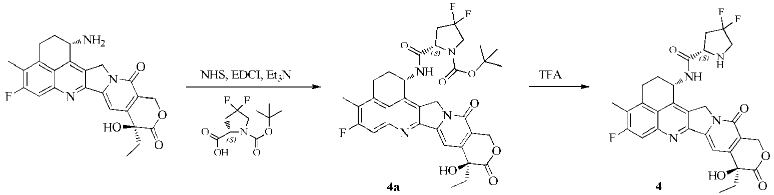

Under ice-bath cooling, DCM (30 mL), N-Boc-4,4-difluoroproline (1.78 g, 7.05 mmol), EDC hydrochloride (1.35 g, 7.05 mmol) and NHS (811.92 mg, 7.05 mmol) were added to a reaction flask. The reaction mixture was stirred under ice-bath for 5 minutes, followed by stirring at room temperature for 2 hours. A solution of exatecan mesylate (2.5 g, 4.70 mmol) in DMF (30 mL) and triethylamine (713.87 mg, 7.05 mmol) was added slowly. The mixture was stirred continuously at room temperature overnight. Water was added, and the mixture was extracted with DCM, purified by silica gel column chromatography to afford compound **4a** as a white solid, N-Boc-4,4-difluoroprolyl exatecan (2.8 g, yield 87%, HPLC 98%); LCMS: (M+1)⁺ 669.17 (calculated value: 668.25).

The above-mentioned Boc-compound **4a** (2.8 g, 4.10 mmol) was taken and dissolved in DCM (25 mL). TFA (8.94 g, 78.41 mmol) was added, and the mixture was stirred at room temperature overnight. The solvent was removed under reduced pressure, and acetonitrile was added. Compound **4** was precipitated as a yellow solid, 4',4'-difluoroprolyl exatecan (2.3 g, yield 81%, HPLC 98.5%); ¹H NMR (600 MHz, DMSO-*d*₆) δ 9.15 (d, *J*=8.7 Hz, 3H), 7.78 (d, *J*=10.8 Hz, 1H), 7.29 (s, 1H), 6.55 (s, 1H), 5.62 (dt, *J*=8.8, 4.4 Hz, 1H), 5.42 (s, 2H), 5.28-5.07 (m, 2H), 4.52 (t, *J*=8.6 Hz, 1H), 3.76 (td, *J*=12.2, 7.6 Hz, 3H), 3.18 (qt, *J*=17.1, 8.4 Hz, 2H), 2.82 (tt, *J*=14.6, 8.8 Hz, 1H), 2.61 (qd, *J*=14.7, 8.8 Hz, 1H), 2.43-2.30 (m, 3H), 2.29-2.13 (m, 2H), 1.85 (dh, *J*=21.6, 7.2 Hz, 2H), 0.87 (t, *J*=7.3 Hz, 3H); ¹³C NMR (151 MHz, DMSO-*d*₆) δ 172.84, 163.02, 162.64, 161.37, 157.15, 152.86, 150.44, 148.54, 145.56, 138.57, 136.44, 126.55, 124.49, 121.68, 119.81, 110.61, 97.33, 72.80, 65.71, 49.49, 45.24, 40.39, 40.25, 40.11, 39.97, 39.83, 39.69, 39.55, 30.81, 27.24, 22.61, 11.43, 11.40, 8.23; LCMS: m/z [M+H]⁺ 569.24 (calculated value 568.19).

### Example 5: 2',2'-difluoroacetyl exatecan (5)

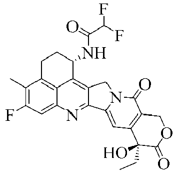

In 10 mL of DMF, exatecan mesylate (500 mg, 0.94 mmol) and TEA (237.96 mg, 2.3 mmol) were added and stirred until dissolved. Difluoroacetic anhydride (163.72 mg, 0.94 mmol) was added dropwise, and the mixture was stirred at room temperature for 60 min. The solvent was removed under reduced pressure, and purified by silica gel column chromatography to afford the product 2',2'-difluoroacetyl exatecan (420 mg, yield 87%, HPLC 99%); ¹H NMR (600 MHz, DMSO-*d*₆) δ 9.43 (d, *J*=8.4 Hz, 1H), 7.82 (d, *J*=10.8 Hz, 1H), 7.32 (s, 1H), 6.53 (s, 1H), 6.25 (s, 0H), 5.62 (dt, *J*=9.1, 4.9 Hz, 1H), 5.43 (s, 2H), 5.27 (s, 1H), 5.17 (s, 1H), 3.18 (p, *J*=6.0 Hz, 2H), 2.41 (s, 3H), 2.25 (dq, *J*=15.3, 5.2 Hz, 1H), 2.18 (ddt, *J*=14.0, 9.9, 5.0 Hz, 1H), 1.87 (dtt, *J*=21.4, 14.4, 7.3 Hz, 2H), 0.87 (t, *J*=7.3 Hz, 3H); LCMS: (M+1)⁺ 514.25 (calculated value : 513.47).

### Example 6: 2',2'-difluoropropionyl exatecan (6)

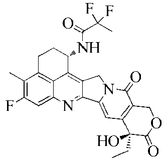

2,2-Difluoropropanoic acid (50 mg, 0.45 mmol) was dissolved in DMF (2 mL), and DIPEA (59 mg, 0.46 mmol) was added. The mixture was stirred until clear. DIC (58 mg, 0.45 mmol) and HATU (171 mg, 0.45 mmol) were added sequentially, and the mixture was stirred at room temperature for 30 minutes. Exatecan mesylate (239 mg, 0.45 mmol) was added, and the mixture was stirred continuously at room temperature for 3 hours. Purification was performed by reversed-phase column chromatography using a C18 spherical column (20-35 µm, 100 Å, 40 g). Acetonitrile and 0.05% aqueous TFA were used as mobile phases B2 and A2, respectively (the mobile phase ratio was changed from A2:B2 = 95:5 to A2:B2 = 61:39), then, the acetonitrile ratio was maintained at 39% and elution was continued for 30 min to afford product 6 as a yellow solid, 2',2'-difluoropropionyl exatecan (135 mg, yield 57%); ¹H NMR (600 MHz, DMSO-*d*₆) δ 9.40 (d, *J*=8.6 Hz, 1H), 7.78 (d, *J*=10.9 Hz, 1H), 7.30 (s, 1H), 6.52 (s, 1H), 5.60 (dt, *J*=8.5, 5.9 Hz, 1H), 5.41 (s, 2H), 5.15 (s, 1H), 5.09 (s, 1H), 3.22 (dt, *J*=16.8, 6.3 Hz, 1H), 3.15 (dt, *J*=16.7, 6.3 Hz, 1H), 2.39 (d, *J*=1.7 Hz, 3H), 2.21 (q, *J*=6.4 Hz, 2H), 1.92-1.79 (m, 5H), 0.87 (t, *J*=7.3 Hz, 3H); LCMS: [M+1]⁺ 528.50 (calculated value : 527.17).

### Example 7: Tumor cell growth inhibitory activity

Human esophageal cancer cells OE33, human breast cancer cells MDA-MB-231, human gastric cancer cells NCI-N87, human lung cancer cells NCI-H1975, human esophageal cancer cells TE12, human breast cancer cells SK-BR-3, etc., were cultured in RPMI1640 (Cellmax) supplemented with 10% fetal bovine serum (Cellmax). Tumor cells in the exponential growth phase were diluted to a concentration of 1×10⁵ cells/mL with culture medium and added to 96-well cell culture plates at 100 µL per well, then returned and incubated overnight in a 37°C incubator with 5% CO₂. On the following day, the compounds were diluted to concentrations of 10000 nM, 2000 nM, 400 nM, 80 nM, 16 nM, 3.2 nM, 0.64 nM, and 0.13 nM with culture medium, and the diluted compounds were added to the 96-well cell culture plates at 2 µL per well, with three replicate wells for each concentration. Negative control and blank control groups without addition of compounds were added with 2 µL of the dilution liquid per well. After the addition of the compounds, the plates were returned to the 37°C incubator with 5% CO₂ to continue the incubation for 72 h. After the incubation, the cell culture plates were removed, and the culture medium in the plates was aspirated using a pipette. Then, 100 µL of culture medium containing 10% CCK-8 was added to each well, and the plates were incubated at 37°C for 3 h. After the incubation, the plates were removed, kept in the dark, and placed in an ELISA plate. Absorbance was measured at 630 nm as the reference wavelength and 450 nm as the test wavelength. Based on the absorbance values, the IC₅₀ values (Table 1) were calculated using the four-parameter logistic regression method in GraphPad.

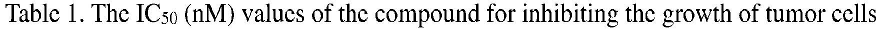

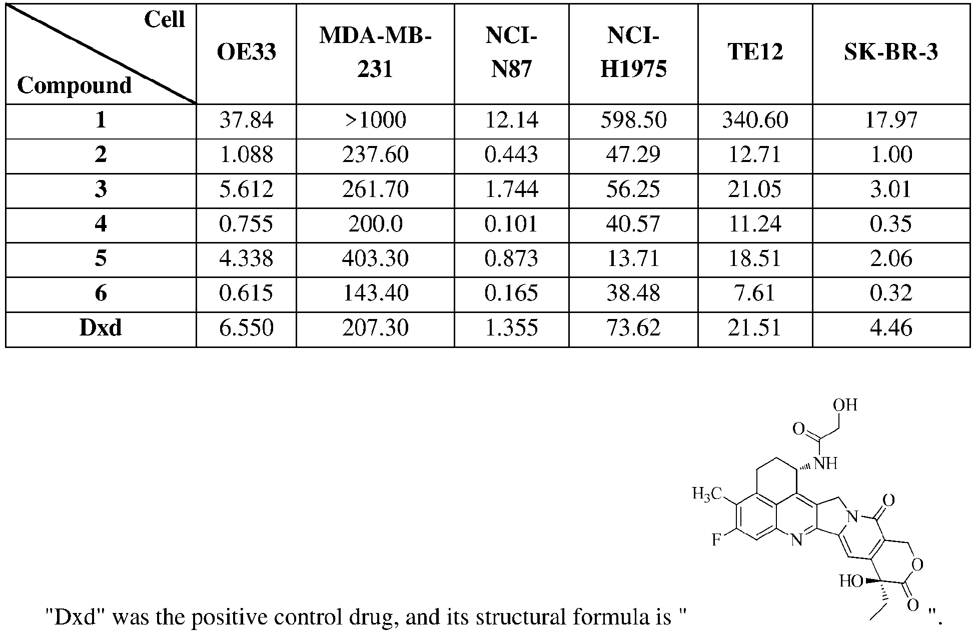

The Example compounds provided herein exhibit good inhibitory effects on the growth of tumor cells. The IC₅₀ values of some compounds for inhibiting tumor cell growth can reach below 10 nM, or even below 1 nM, demonstrating significant anti-cancer activity.

The foregoing descriptions of specific exemplary embodiments of the present disclosure have been presented for purposes of illustration and example. These descriptions are not intended to limit the present disclosure to the precise forms disclosed, and obviously many modifications and variations are possible in light of the above-mentioned teachings. The exemplary embodiments were chosen and described in order to explain the particular principles of the present disclosure and its practical application, to thereby enable those skilled in the art to make and use various exemplary embodiments of the present disclosure and various alternatives and modifications. It is intended that the scope of the present disclosure be defined by the claims and their equivalents.

## Claims

1. A compound of formula (I), or a pharmaceutically acceptable salt, a stereoisomer, or a prodrug thereof: 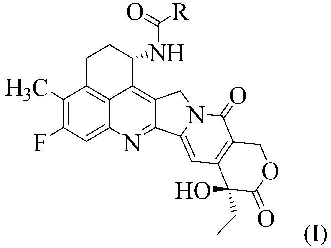
in the Formula, R is selected from any one of C₁~C₆ alkyl, halogen-substituted C₁~C₆ alkyl, 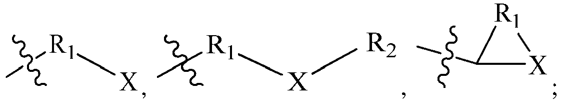
wherein, R₁ is selected from C₁~C₆ alkylene, the C₁~C₆ alkylene is optionally substituted with one or more substituents selected from halogen, C₁~C₆ alkyl or halogen-substituted C₁~C₆ alkyl;
R₂ is selected from any one of halogen, C₁~C₆ alkyl or halogen-substituted C₁~C₆ alkyl; X is selected from any one of amino, imino.

2. The compound, or pharmaceutically acceptable salt, stereoisomer, or prodrug thereof according to claim 1, wherein, R₁ is selected from methylene, halogen-substituted methylene or trifluoromethyl-substituted methylene;
preferably, R₁ is selected from -CHF-, -CHF₂-, -CH₂- or -CH(CF₃)-.

3. The compound, or pharmaceutically acceptable salt, stereoisomer, or prodrug thereof according to claim 1 or 2, wherein, R₂ is selected from any one of halogen, methyl, methylene, ethyl, propyl, isopropyl, butyl, isobutyl, pentyl, isopentyl, hexyl, heptyl; preferably, any one of methylene, methyl, ethyl, isopropyl;
the halogen is selected from any one of -F, -Cl, -Br, -I, preferably -F.

4. The compound, or pharmaceutically acceptable salt, stereoisomer, or prodrug thereof according to any one of claims 1~3, wherein, X is selected from -NH- or -NH₂.

5. The compound, or pharmaceutically acceptable salt, stereoisomer, or prodrug thereof according to any one of claims 1~4, wherein, 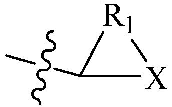 is selected from 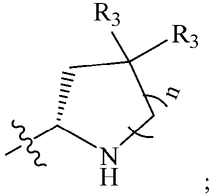
wherein, R₁ is selected from halogen; preferably F;
n is selected from 1, 2, 3 or 4, preferably, n is 1;
preferably, R is selected from

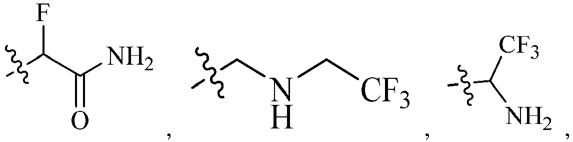

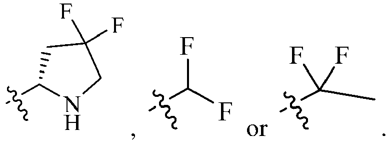

6. The compound, or pharmaceutically acceptable salt, stereoisomer, or prodrug thereof according to any one of claims 1~5, wherein, the compound of formula (I) is any one of the following compounds:

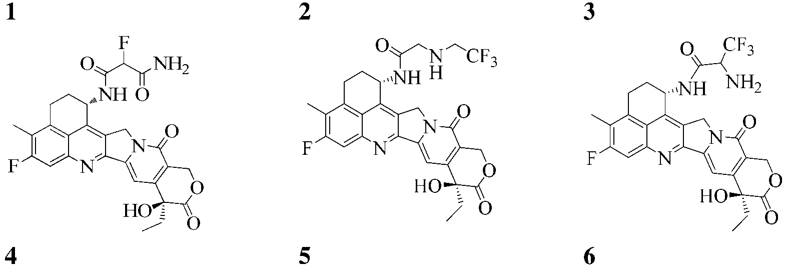

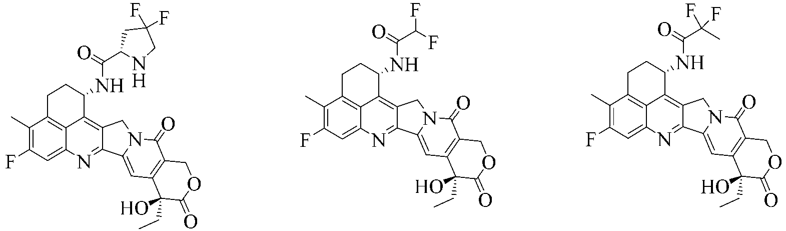

7. A method for preparing the compound according to any one of claims 1~6, comprising:
reacting halogen-substituted carboxylic acid, carboxylic anhydride, or acyl chloride with exatecan to prepare the compound according to any one of claims 1~6;
preferably, the halogen-substituted carboxylic acid, carboxylic anhydride, or acyl chloride is selected from any one of 2-fluoromalonic acid, 2-amino-3,3,3-trifluoropropanoic acid, difluoroacetic anhydride, 2,2-difluoropropanoic acid.

8. An antibody-drug conjugate, comprising a small molecule drug, a linker, and an antibody, wherein, the small molecule drug comprises the compound, or pharmaceutically acceptable salt, stereoisomer, or prodrug thereof according to any one of claims 1~6.

9. A pharmaceutical composition, comprising the compound, or pharmaceutically acceptable salt, stereoisomer, or prodrug thereof according to any one of claims 1~6, or the antibody-drug conjugate according to claim 8.

10. Use of the compound, or pharmaceutically acceptable salt, stereoisomer, or prodrug thereof according to any one of claims 1~6, the compound afforded by the preparation method according to claim 7, the antibody-drug conjugate according to claim 8, or the pharmaceutical composition according to claim 9, in the manufacture of a medicament for treating a cancer; preferably, the cancer comprises any one of gastric cancer, esophageal cancer, esophagus cancer, breast cancer, breast adenocarcinoma, lung cancer.

11. A method for treating a cancer, comprising the step of administering to a patient in need thereof the compound, or pharmaceutically acceptable salt, stereoisomer, or prodrug thereof according to any one of claims 1~6, the compound afforded by the preparation method according to claim 7, the antibody-drug conjugate according to claim 8, or the pharmaceutical composition according to claim 9;
preferably, the cancer comprises any one of gastric cancer, esophageal cancer, esophagus cancer, breast cancer, breast adenocarcinoma, lung cancer.
